(19) Europäisches Patentamt / European Patent Office / Office européen des brevets

(11) **EP 3 730 060 A1**

(12) **EUROPEAN PATENT APPLICATION**

(43) Date of publication:
28.10.2020 Bulletin 2020/44

(51) Int Cl.:
*A61B 8/02* (2006.01)   *A61B 8/08* (2006.01)

(21) Application number: **19170882.5**

(22) Date of filing: **24.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **FRANCK, Christoph Florian**
  **5656 AE Eindhoven (NL)**
• **WOHLSCHLAGER, Markus Silvester**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **FETAL ULTRASOUND PROCESSING UNIT FOR SEPARATING HEART RATE SIGNALS**

(57)    A processing unit and method are provided for processing fetal Doppler ultrasound data to extract a set of signals representative of different distinct heart rate signal sources, e.g. for detecting two fetal heart rate sources (22a, 22b) in a twin pregnancy. Doppler data is received from a plurality of different transducer sources (12a, 12b), corresponding to different (but potentially overlapping) tissue regions within the maternal abdomen. From the multiple sources of Doppler ultrasound data a single set of input signal channels is compiled (53), each corresponding to a different tissue region within the maternal abdomen. These are then processed successively by a PCA algorithm (54) followed by an ICA algorithm (58), which work to unmix the multiple heart rate sources present in each of the input channels, and derive a set of output signals from the ICA which can be taken as representative of the separate heart rate sources (22a, 22b).

FIG. 5

EP 3 730 060 A1

**Description**

FIELD OF THE INVENTION

The invention provides an ultrasound processing unit for use in distinguishing different heart rate signal sources within ultrasound Doppler data.

BACKGROUND OF THE INVENTION

Electronic fetal monitoring (EFM) typically uses Doppler ultrasound to acquire a pulse (heart rate) signal from a fetus in utero during pregnancy and labor. The fetal heart rate (FHR) is calculated using the acquired pulse signal.

The Ultrasound (US) Doppler transducer used for this purpose typically utilizes an unfocused, approximately cylindrical ultrasound beam field. An extent of the beam volume is defined by a characteristic reception time window. During this window the US transducer is set to acquire reflected signals from any moving anatomical structures.

Typically, EFM requires as many ultrasound transducer units positioned on the maternal abdomen as there are fetuses: monitoring a twin pregnancy requires two transducer units, monitoring a triple pregnancy requires three, and so on. Current EFM systems require careful placement of the transducer units, so that the ultrasound beam of each transducer unit only covers one fetal pulse rate source like the heart of the fetus or large fetal arteries, and no maternal pulse rate source, such as large abdominal arteries. If this requirement is not met and multiple independent pulse rate sources are present in the volume covered by the ultrasound beam of a transducer, the EFM system may be unable to calculate the fetal heart rate or may calculate an erroneous heart rate.

In particular, if the volume covered by the ultrasound beam of a single transducer unit includes multiple heart rate sources, a Doppler ultrasound signal may be acquired which is a mixture of two or more pulse rate sources. As a result, the FHR (fetal heart rate) calculation performed on the basis of this signal may fail or lead to an erroneous measurement.

Thus great care is currently required when placing an ultrasound transducer unit on the maternal abdomen to ensure that each transducer unit only picks up one individual pulse rate source in its ultrasound beam field.

Correct positioning consumes time and may often require multiple attempts. Repositioning the transducer units may also become necessary during a scan if the position of any fetus changes.

The approach currently used for known EFM systems is that when multiple ultrasound transducer units are used, each transducer unit records a separate Doppler ultrasound signal acquired from its own ultrasound beam field and calculates a single pulse rate from it. The pulse rate is then transmitted to a central unit of the EFM system for display and recording.

This approach assumes therefore the ultrasound signal acquired by each separate transducer unit relates to just a single heart rate source, and includes no provision to address or remedy the possible case of multiple heart rate sources being present in a single acquired signal. It also does not allow for the possibility of crossing beam fields, and thus the possibility that two transducer units capture signals relating to one or more of the same heart rate sources.

Thus, in addition to significant time consumption in ensuring correct placement of each separate ultrasound transducer unit, errors in heart rate calculation can also be a frequent occurrence due to errors in positioning and accidental inclusion of multiple heart rate sources in a single US beam field.

There would therefore be advantage in providing an improved approach to identifying and distinguishing different unique heart rate sources present within a plurality of US signals acquired by a plurality of different US transducer units.

SUMMARY OF THE INVENTION

The invention is defined by the claims.

According to examples in accordance with an aspect of the invention, there is provided an ultrasound processing unit, for use in fetal monitoring for distinguishing different heart rate sources within received Doppler ultrasound data,

the ultrasound processing unit being communicatively coupleable in use with at least two ultrasound transducer units;

and the ultrasound processing unit configured to:

receive first input Doppler ultrasound data from a first ultrasound transducer source, and receive second input Doppler ultrasound data from a second ultrasound transducer source;

compile from the first and second input Doppler ultrasound data a single set of input ultrasound signal channels, each corresponding to a different particular tissue region within the subject;

perform a principal component analysis, PCA, procedure, configured to identify one or more linear combinations of the input signal channels which are statistically uncorrelated, the linear combinations defining, when composed, a set of first output signals, and

perform an independent component analysis, ICA, procedure, configured to identify one or more linear combinations of said first output signals which are statistically independent from one another, said one or more linear combinations defining a set of one or more second output signals. Due to the effect of the combined PCA and ICA procedures, the resulting second output signals can each reliably be taken as corresponding to a single heart rate signal source.

Thus the one or more second output signals provide signals corresponding to distinct heart rate sources.

Embodiments of the present invention thus propose a different approach to treatment of multiple transducer signals to that known from current EFM systems. Instead of processing and deriving one separate heart rate signal for each separate transducer unit, the invention is based on collecting the ultrasound signal channel(s) recorded by a plurality (at least two) of transducer units at a central point (i.e. the ultrasound processing unit), collating the recorded signals channels into a single combined set, and mathematically processing these to identify and extract the individual underlying heart rate signals present within them.

In particular, embodiments are based on using the mathematical techniques of principal component analysis (PCA) and independent component analysis (ICA) to distinguish and separate independent heart rate sources which may be present as mixtures in the original ultrasound signal channels.

Thus, presence of multiple heart rate sources within the ultrasound data from each transducer unit can be effectively handled, and errors in fetal heart rate calculation due to multiple source pickup are substantially avoided.

This therefore allows for simplified transducer placement and increases the reliability and availability of the heart rates (fetal and maternal) recorded and displayed by the EFM system.

Embodiments of the invention thus reduce the time and effort necessary for correct transducer placement.

The processing unit may receive from each of the first and second ultrasound transducer sources ultrasound data in the form of one or more ultrasound signal (channels). The compiling of the single set of input signal channels may in this case comprise simply collating these received channels together into a single group.

Alternatively, the processing unit may receive ultrasound data from each of the first and second transducer source which has not been processed to extract separate ultrasound signals. In this case, the ultrasound processing unit may extract from each of the first and second input Doppler ultrasound data one or more ultrasound signal channels and compile or collate these into said single set of input signal channels. This extraction of signal channels may comprise for instance a process of gating a given input ultrasound signal over a series of temporally successive windows, each window providing a different channel. The resulting channels may each contain a mixture of the multiple heart rate sources.

Accordingly, extraction of the separate ultrasound signal channels included in the single set of input signal channels may be performed by the ultrasound processing unit or may be performed externally to the provided ultrasound processing unit, for instance by the source ultrasound transducer unit.

Each of the compiled set of input ultrasound signal channels correspond to a different particular tissue region, i.e. the first and second input Doppler ultrasound data are together representative of a plurality of different tissue regions, and the set of signal channels correspond to these different tissue regions.

The different particular tissue regions means regions within the subject which are not exactly spatially coincident with one another. The different particular tissue regions may overlap. The different tissue regions may include different depth regions, or regions at approximately the same depth but laterally displaced from one another. Each of the first and second input Doppler ultrasound data may include data for one or more different tissue regions, for example one or more different depth regions.

The first and second ultrasound transducer source may each be an ultrasound transducer unit comprising one or more ultrasound transducers, for instance an ultrasound probe.

The PCA and ICA algorithms are mathematical techniques used in signal analysis for separating out mixtures of signal sources. By providing a plurality of input mixed signals (the input signal channels), the combination of these algorithms enables extraction of a set of second output signals, each of which corresponds to a single heart rate source.

The combined application of the PCA procedure and ICA procedure leads to more complete unmixing of the original heart rate sources present within the data than would be achievable with either of the two processes alone. As a result, the set of second output signals can be taken to be reliably representative of different heart rate sources.

Each linear combination of input signals identified by the PCA procedure corresponds to a single first output signal. The PCA procedure may identify the linear coefficients (or weightings) which define each linear combination, and/or may generate each first output signal by combining the relevant signals which form each one, with the weightings identified.

The PCA procedure is configured to identify one or more linear combinations of the input signal channels which are statistically uncorrelated with one another.

Statistical correlation is a term of the art. In particular, statistical correlatedness is a well-defined term in the field of mathematical statistics. Two random variables X and Y are statistically uncorrelated if the expected value of their product is equal to the product of their expected values:

$$E\{X \cdot Y\} = E\{X\} \cdot E\{Y\}$$

In the context of the present invention, the variable X would be a first one of the identified linear combinations of input signal channels, and the variable Y would be a second one of the identified linear combinations of input signal channels.

This definition is readily expanded to vectors of random variables. In particular, the first term in the equation above becomes the expectation value of the outer, or dyadic, product of the random vectors x and y and is the cross-correlation matrix of the two vectors:

$$E(\mathbf{x} \cdot \mathbf{y}^T) = E(\mathbf{x}) \cdot E(\mathbf{y}^T)$$

For the vector version, the expected value of the outer product of vectors x and y must be equal to the outer product of their individual expected values for the vectors to be uncorrelated. The outer product of two vectors is a matrix. The superscript $^T$ denotes a transpose of the relevant matrix in the above expression.

In the context of the present invention, the PCA algorithm may generate as an output a single vector, **z**, whose elements are constituted by the set of first output signals (the set of linear combinations of input signal channels). The aim of the PCA algorithm, as discussed, is to make the individual components (channels) of such a (single) output vector uncorrelated.

To apply the above (general) uncorrelatedness condition for a single output vector to be generated by the PCA algorithm, the condition may be expressed in slightly different form. Uncorrelatedness of the individual elements of a single output vector, z, of output signal channels is achieved when all elements outside the diagonal of the covariance matrix

$$C_z = E((\mathbf{z} - \mathbf{m_z}) \cdot (\mathbf{z} - \mathbf{m_z})^T)$$

are zero (where $\mathbf{m_z}$ is the vector of mean values of the elements of z). The diagonal elements are nonzero for any component that does not have a constant value, as the diagonal elements contain the variances of each channel.

The definition of statistical correlatedness may be found for example in the book: Appo Hyvärinen, Juha Karhunen, Erkki Oja, "Independent Component Analysis", John Wiley & Sons, Inc., 2001.

Statistical independence is also a well-defined term in mathematical statistics. Two variables, x, y, are statistically independent if their joint probability (the probability of observing certain combinations of values) factorizes into a product of individual probabilities:

$$p_{x,y}(x,y) = p_x(x) \cdot p_y(y)$$

This effectively means that knowledge of the value of one of the variables x, y, gives no information at all about the value of the other variable.

Statistically independent variables are always uncorrelated, but uncorrelated variables are not necessarily independent. By way of example, consider two random variables where one variable is always zero if the other variable is different from zero. The two variables are uncorrelated, as their product is always equal to zero, but not independent, as knowing that one variable is nonzero allows the deduction that the other variable is zero.

According to one or more embodiments, the compiled single set of ultrasound signal channels may comprise channels corresponding to at least two different depth regions within the subject. One or both of the first and second input Doppler ultrasound data may include data corresponding to a plurality of different depth regions.

The compiled single set of ultrasound signal channels may comprise channels corresponding to at least two different lateral spatial regions within the subject. By this is meant regions laterally displaced from one another (i.e. in a width direction, perpendicular to a depth direction). For example, where beamforming is used to enable ultrasound beams to be generated in multiple different directions through the subject, the different lateral regions may be different beam directional regions (e.g. tubular beam regions extending in different directions into the subject from the tissue surface).

The PCA procedure may be configured to identify the linear combinations of said input signal channels that result in first output signals having a combined signal strength or variance exceeding a defined threshold while being statistically uncorrelated with one another.

The threshold may be defined relative to an average or a maximum signal strength among the input signals, or among possible combined signals. For example, the threshold might be a signal strength 50% or 75% of the maximum signal strength among the possible combined signals.

The PCA algorithm thus selects the linear combinations which result in a largest combined signal strength, while remaining statistically uncorrelated.

According to advantageous embodiments, the processing unit may be further configured to generate the set of second output signals in accordance with the identified linear combinations. This comprises combining the input signals together with the particular sets of weightings defined for each linear combination (each second output signal). Thus signals for each of the independent heart rate sources are reconstructed, and may be output, for example for display or other communication to a user, or for further processing.

By reconstructing a pulse signal for each identified separate source, these embodiments additionally aim to avoid possible "quiet" periods which can occur in generated pulse signals in known EFM systems, in which there are times of missing fetal heart rates, or times of erroneous fetal heart rates.

Signal combination is a routine process and the skilled person will be aware of means for achieving this.

The processing unit may be adapted to process the second output signals to derive from each a heart rate signal or heart rate measurement.

The processing unit may be further adapted to attribute to each of the second output signals a physiological source.

The processing unit here derives a physiological attribution for each of the second output signals by determining a physiological source of each signal.

The processing unit may determine for instance which of the output signals corresponds to the maternal heart rate and which to the fetal heart rate(s).

The attribution may be based on comparison of one or more signal characteristics of the second output signals.

According to one or more embodiments, the compiling may comprise compiling from the first and second input Doppler ultrasound data a single vector of input ultrasound signal channels, each corresponding to a different particular tissue region within the subject, i.e. the generated single set is a single vector.

The PCA procedure is configured in general to provide as an input to the ICA procedure an indication of the identified linear combinations of the input signals.

In some examples, the PCA procedure may provide as an input to the ICA procedure a plurality of sets of linear coefficients which define said identified linear combinations of input signals. The PCA procedure may output a matrix, the elements of which are populated by the linear coefficients.

Additionally or alternatively, the PCA procedure may be configured to generate the set of first output signals in accordance with the identified linear combinations, and provide the signals as an input to the ICA procedure.

As noted above, according to one or more embodiments, compiling the set of input signal channels may comprise extracting the signal channels from source data. This may comprise gating the input Doppler ultrasound data over a plurality of different temporal windows, for example over a series of temporally successive windows. The input signal channels may hence each correspond to a different captured ultrasound window.

Examples in accordance with a further aspect of the invention provide an ultrasound apparatus comprising:

an ultrasound processing unit in accordance with any example or embodiment outlined above or described below, or in accordance with any claim of this application; and
one or more ultrasound transducers, operatively coupled to the ultrasound processing unit, for providing at least one of the first and second input Doppler ultrasound data to the ultrasound processing unit.

The ultrasound apparatus may comprise an ultrasound probe unit, the probe unit incorporating the ultrasound processing unit and at least a portion of the one or more ultrasound transducers.

The probe unit may for example have a housing, the ultrasound processing unit and the one or more ultrasound transducers being included within the housing.

The connection interface may be a wired connector, or may be a wireless connection interface for connecting to a wireless ultrasound probe.

Examples in accordance with a further aspect of the invention provide a patient monitoring system comprising:

an ultrasound processing unit in accordance with any example or embodiment outlined above or described below, or in accordance with any claim of this application; and
a connection interface for connecting in use to at least two ultrasound transducer units.

The patient monitoring system may further comprise comprising a set of at least two ultrasound transducer units coupled to said connection interface. The transducer units may be ultrasound probes for example.

One or both of the ultrasound transducer units may be only a transmit/receive unit, i.e. comprising one or more ultrasound transducers for transmitting and sensing ultrasound signals. Here, the ultrasound processing unit comprises all signal processing components, including components for digitization and demodulation of the analogue signals output by the transducer unit, to separate the different signal channels for different tissue regions. In this case, analogue signals are communicated from each transducer unit to the ultrasound processing unit via the communication interface.

In other examples, the ultrasound transducer unit may additionally comprise local, or on-site, signal processing components for performing digitization and demodulation of the signals and to achieve separation of the signal channels for different tissue regions. In this case, the resulting digital data representative of the separated signal channels is communicated from the ultrasound transducer unit to the ultrasound processing unit.

The patient monitoring system may include a base station or base unit, to which at least one or more ultrasound transducer units, e.g. ultrasound probes, can be connected. The base station may include a display for displaying results of the processing performed by the ultrasound processing unit.

The ultrasound processing unit may be comprised by the base station. Alternatively, the ultrasound processing unit may in some examples be comprised by the ultrasound transducer unit.

The patient monitoring system may include a base station connected or connectable with an ultrasound apparatus as described above, comprising an ultrasound probe integrating ultrasound transducers and the ultrasound processing unit.

The patient monitoring system may further include a controller adapted to control acquisition of ultrasound data by one or more connected transducer units in use.

The controller may control transmit and receive circuits of each of the one or more ultrasound transducer units to acquire the ultrasound signals representative of different tissue regions, e.g. different depths. The controller may control durations of, and timings between, transmit pulses and receive windows. The controller may control gating of the input Doppler signal data over defined time windows to thereby separate different input signal channels corresponding to different depths within the subject's tissue.

In certain examples, the different depths may correspond to different depth ranges within a single generated cylindrical ultrasound beam field.

Examples in accordance with a further aspect of the invention provide an ultrasound processing method for use in distinguishing different heart rate sources within received Doppler ultrasound data, the method comprising

receiving first input Doppler ultrasound data from a first ultrasound transducer source, and receiving second input Doppler ultrasound data from a second ultrasound transducer source;

compiling from the first and second input Doppler ultrasound data a single set of input ultrasound signal channels, each corresponding to a different particular tissue region within the subject;

performing a principal component analysis, PCA, procedure, configured to identify one or more linear combinations of the input signal channels which are statistically uncorrelated, the linear combinations defining, when composed, a set of first output signals, and

performing an independent component analysis, ICA, procedure, configured to identify one or more linear combinations of said first output signals which are statistically independent from one another, said one or more linear combinations defining a set of one or more second output signals. The one or more second output signals provide signals which can be reliably taken as corresponding to distinct heart rate sources.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a known approach for acquiring fetal heart rate data using multiple ultrasound transducer units based on positioning the probes so as to capture only a single heart rate source in each transducer unit beam;

Fig. 2 illustrates an example positioning error in the known approach of Fig. 1, leading to erroneous heart rate calculation;

Fig. 3 is a block diagram of steps performed by an example ultrasound processing unit according to one or more embodiments;

Fig. 4 shows a workflow of an example ultrasound processing unit according to one or more embodiments.

Fig. 5 illustrates use of an example embodiment of the invention;

Fig. 6 illustrates further use of an example embodiment of the invention; and

Fig. 7 shows an example ultrasound system according to one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processing unit and method for processing fetal Doppler ultrasound data to extract a set of signals representative of different distinct heart rate signal sources, i.e. maternal heart rate and fetal heart rate. Doppler data is received from a plurality of different transducer sources, corresponding to different (but potentially overlapping) tissue regions within the maternal abdomen. From the multiple sources of Doppler ultrasound data is compiled a single set of input signal channels, each corresponding to a different tissue region within the maternal abdomen. These are then processed successively by a PCA algorithm followed by an ICA algorithm, which work to un-mix the multiple heart rate sources present in each of the input channels, and derive a set of output signals from the ICA which can be taken as representative of separate heart rate sources.

As discussed above, state of the art electrical fetal monitoring (EFM) systems do not deal satisfactorily with cases in which multiple heart rate sources are present within the maternal abdomen (e.g. multiple pregnancy cases). Firstly, in these cases, typically EFM requires as many ultrasound transducer units positioned on the maternal abdomen as there are fetuses. In addition, current EFM systems require very careful placement of the transducers to ensure that the ultrasound beam of each transducer unit covers only one heart rate source.

The approach currently used for known EFM systems is that when multiple ultrasound transducer units are used, each transducer unit records a separate Doppler ultrasound signal acquired from its own ultrasound beam field and calculates a single pulse rate from it. The pulse rate is then transmitted to a main unit of the EFM system for display and recording.

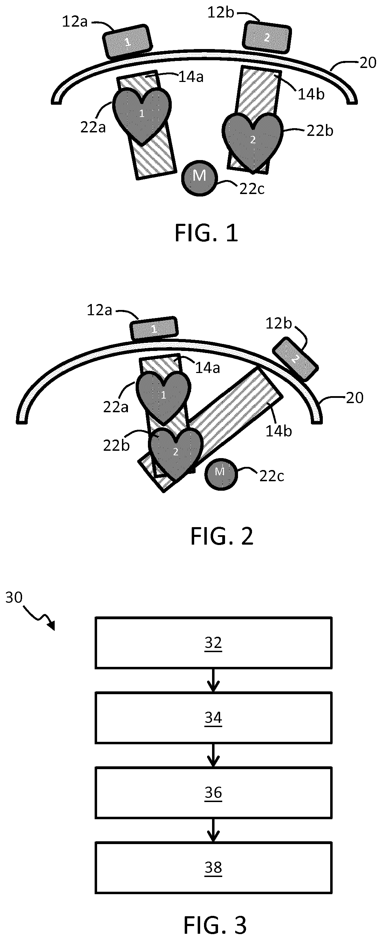

This is illustrated schematically in Fig. 1 which shows a first 12a and second 12b ultrasound transducer unit positioned on the skin surface of the maternal abdomen 20. These transmit a first 14a and second 14b ultrasound beam field respectively into the maternal abdomen for sensing heart rate sources. Two fetuses are assumed to be present in this example, so that there is a first 22a, and second 22b fetal heart rate source present within the abdomen, in addition to the maternal heart rate source 22c. In order to correctly record the two fetal heart rates, each transducer unit 12a, 12b must be carefully positioned such that each of the ultrasound beam fields 14a, 14b contains only a single heart rate signal source 22a, 22b. The maternal pulse rate source 22c (e.g. the abdominal artery) is not covered by either ultrasound beam field 14a, 14b).

This approach assumes therefore the ultrasound signal acquired by each separate transducer unit relates to just a single heart rate source, and includes no provision to address or remedy the possible case of multiple heart rate sources being present in a single signal. It also does not allow for the possibility of crossing beam fields, and thus the possibility that two transducer units capture signals relating to one or more of the same heart rate sources.

This approach is time-consuming and limits options for transducer placement. It may also require repositioning of the transducer unit(s) if the fetus(es) change position.

Errors in positioning of the transducer units can also occur. This is illustrated schematically in Fig. 2. Here, the ultrasound transducer units 12a, 12b have been inadvertently positioned such that their respective ultrasound beam fields 14a, 14b cross. The first transducer unit 12a has both the first 22a and second 22b heart fetal rate source in its ultrasound beam field 14a, and the second transducer unit 12b has just the second heart rate source 22b in its ultrasound beam field 14b. As a result, the first ultrasound transducer unit 12a will pick up a mixture of heart rate signal sources. With known technology, this may lead either to an erroneous heart rate calculation result for the first transducer unit, or no heart rate calculation at all. In either case, clinical decision making will be affected, with potentially negative consequences.

Furthermore, as in the example of Fig. 1, no transducer covers the maternal pulse rate source 22c in its ultrasound field of view 14a, 14b.

In addition, due to the discussed potential problems of multiple source pickup in known technology, operators often are required to keep the beam width area relatively small in order to reduce the likelihood of picking up unwanted interference signals. However, a narrow covered beam area has the result that the target (the fetal heart) may move quickly out of focus. As a result, frequent repositioning of the transducer unit is often necessary, which is inconvenient and can disturb the examination process.

Embodiments of the present invention aim, among other things, to make transducer placement in multi-transducer EFM systems easier and to increase the availability and reliability of the fetal heart rates calculated by the EFM system.

In brief, according to embodiments of the present invention, each ultrasound transducer unit of the system records one or more Doppler ultrasound signal channels. Multiple channels may be recorded for instance by partitioning the volume covered by the ultrasound beam of one or more of the transducer units by depth, direction or width. In this way, each channel may correspond to a slightly different tissue region. This can be achieved for instance through appropriately controlled gating of the received Doppler signals, or for example through beam-forming (e.g. through delay and sum approaches) where an ultrasound array is used.

The signal channel(s) of each transducer are collected at one collection point (i.e. the ultrasound processing unit), which may be included for instance in one of the transducer units or for example in a central base station of the EFM system. The Doppler ultrasound channel data can be transmitted by wired or wireless connection.

At the central collection point, the individual Doppler ultrasound signal channels may be combined into a single vector of channels, and the signal processing method Principal Component Analysis (PCA) is used to identify a number of orthogonal linear combinations of the vector elements that, in descending order, contain the greatest signal variance (which corresponds to signal power). These principal components are then used as input data for performing Independent Component Analysis (ICA), which identifies linear combinations of the principal components which are statistically independent of one another. These linear combinations may then be taken as representative of independent heart rate signals.

The fetal and possibly maternal heart rates may then be calculated from the reconstructed source signals, for instance using methods known in the art such as autocorrelation.

According to this method, it is no longer strictly necessary to take steps to avoid including more than one pulse rate source in each ultrasound transducer unit beam field. The process of applying PCA followed by ICA allows for separation and reconstruction the individual heart rate sources from input signals containing mixtures of them. This simplifies the process of transducer placement on the abdomen and increases calculated heart rate reliability. In addition, since multiple pulse rate source pickup can be accommodated, there is no longer any need to keep the beam width area minimally small as in previous technologies. This allows the area covered by the ultrasound beam to be selected larger, avoiding the problems discussed above of the target heart moving frequently out of focus, and the consequent need to frequently reposition the transducer unit.

Examples in accordance with a first aspect of the invention provide an ultrasound processing unit for use in fetal monitoring for distinguishing different heart rate sources within received Doppler ultrasound data. The ultrasound processing unit is communicatively coupleable in use with at least two ultrasound transducer units. It may include a connection interface for connecting with at least two ultrasound transducer sources.

Fig. 3 shows a flow diagram illustrating in block diagram form the basic steps 30 performed by the ultrasound processing unit.

The ultrasound processing unit is configured to receive 32 first input Doppler ultrasound data from a first ultrasound transducer source, and receive second input Doppler ultrasound data from a second ultrasound transducer source. More than two sets of input Doppler data may be received in further examples. Any number m sets of input Doppler ultrasound data may be received from a respective m ultrasound transducer sources.

The ultrasound transducer unit is further configured to compile 34 from the first and second input Doppler ultrasound data a single set of input ultrasound signal channels, each corresponding to a different particular tissue region within the subject.

As noted above, different options are possible for this. The processing unit may receive from each of the first and second ultrasound transducer unit ultrasound data in the form of one or more ultrasound signal (channels). The compiling of the single set of input signal channels may in this case comprise simply collating these received channels together into a single group.

Alternatively, the processing unit may receive ultrasound data from each of the first and second transducer unit which has not been processed to extract separate ultrasound signals. In this case, the ultrasound processing unit may extract from each of the first and second input Doppler ultrasound data one or more ultrasound signal channels and compile or collate these into said single set of input signal channels. This extraction of signal channels may comprise for instance a process of gating a given input ultrasound signal over a series of temporally successive windows, each window providing a different channel. The resulting channels may each contain a mixture of the multiple heart rate sources.

Accordingly, extraction of the separate ultrasound signal channels included in the single set of input signal channels may be performed by the ultrasound processing unit or may be performed externally to the provided ultrasound processing unit.

The ultrasound processing unit is further configured to perform 36 a principal component analysis, PCA, procedure, configured to identify one or more linear combinations of the input signal channels which are statistically uncorrelated, the linear combinations defining, when composed, a set of first output signals.

The PCA procedure provides an indication of the identified linear combinations of input signals as an input to the ICA. This may be in the form of a set of linear coefficients, or weightings, of the input signals in some examples. The PCA procedure may comprise generating the first output signals based on the derived linear combinations. These may be provided as an input the ICA procedure in some examples, either instead of, or in addition to, the linear coefficients (weightings).

The ultrasound processing unit is configured to subsequently perform 38 an independent component analysis, ICA, procedure, configured to identify one or more linear combinations of said first output signals which are statistically independent from one another, said one or more linear combinations defining a set of one or more second output signals. The set of second output signals are taken as representative of separate distinct pulse rate sources within the probed region. The ICA procedure may generate the set of second output signals in accordance with the identified linear combinations of the first output signals. These may be provided as an output. These may be displayed on an associated display device for example, for observation by a user, e.g. a clinician.

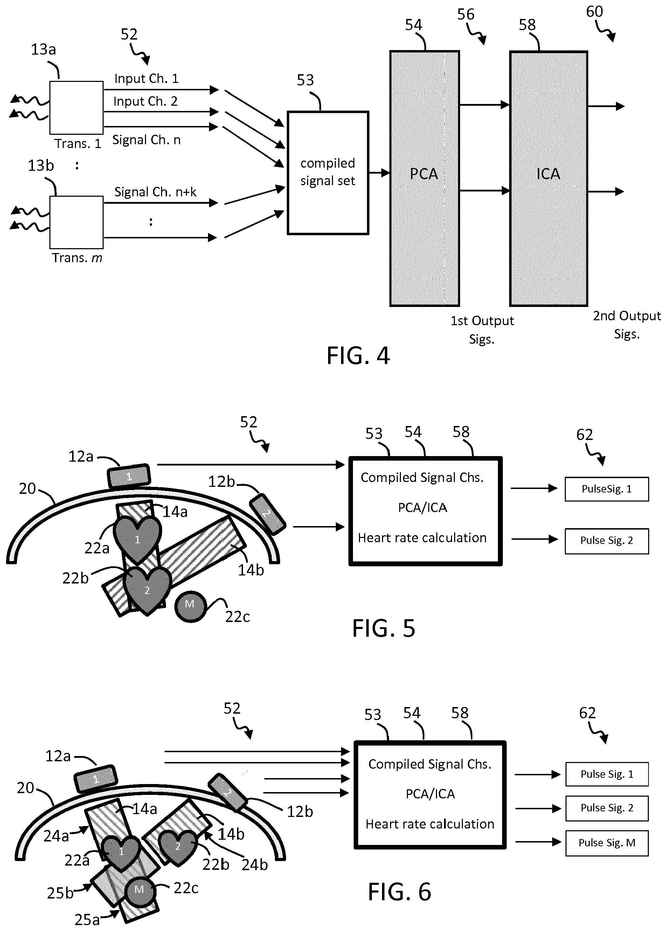

Fig. 4 schematically depicts in more detail an example workflow for an ultrasound processing unit according to one or more embodiments. Although certain functions are shown as performed by separate components, this is for illustration only in this figure. It is to be understood that in general the functionality performed by these components may be performed by a different combination of one or more components, for instance a single unitary processor in some examples.

First and second Doppler ultrasound data is first obtained using a first 13a and second 13b ultrasound transducer unit respectively. Two ultrasound transducer units are shown by way of example, but more than two can be used. Any number, m, of transducer sources can be used to collect a respective m sets of input Doppler ultrasound data.

The Doppler ultrasound signal channel(s) 52 recorded by each transducer unit are transmitted to a central collection device (i.e. the ultrasound processing unit.) This may be located in a central base station, or may be included in one of the ultrasound transducer units. Each transducer unit can record one or more Doppler ultrasound signal channels corresponding to different depth ranges, directions, or beam field widths.

The central collection device combines 53 the input signal channels 52 it receives into one vector of channels. This vector is used as the input for a principal component analysis algorithm 54. This algorithm determines linear combinations (weighted sums) of the input signal channels 52 that capture the largest variance (corresponding to signal power) of the signal while being statistically uncorrelated. The derived set of linear combinations may be compiled into an output vector. The output vector of the PCA algorithm can be used to determine how many heart rate signal sources are present by comparing the variances of the output channels.

However, PCA is typically insufficient alone to achieve complete separation of the independent heart rate source signals. PCA can be used to reduce the total number of channels for further processing steps, as the number of channels produced by the transducer units may be large. Reducing the number of channels to a smaller set of principal component channels that capture a large amount of the variance of the signal simplifies subsequent processing steps, and it excludes mathematical subspaces of the input signal channels that contain mostly noise.

PCA can be performed with a number of different algorithms, including by way of example online machine learning algorithms (such as neural networks) or subspace learning.

The reduced number of principal component channels defines a set of first output signals 56.

These are then used as an input to an independent component analysis (ICA) procedure 58. This technique determines linear combinations of the first output signals 56 that are statistically independent, resulting, in a number of signal channels, each of which may be reliably assumed to contain only the Doppler ultrasound signal of a single heart signal source in the abdomen. The ICA algorithm completes the un-mixing of the pulse signal sources.

The linear combinations derived by the ICA procedure 58 defines a set of second output signals 60.

The second output signals 60 of the ICA procedure 58 may then be used as an input for a heart rate calculation algorithm, e.g. based on autocorrelation. Since each output signal channel 60 is predominantly composed of only one heart rate signal source, erroneous fetal heart rate (FHR) readings due to signal mixtures are much less frequent than with current technology.

In more detail, in operation, ultrasound pulses may be transmitted by a plurality ultrasound receive/transmit units 13a, 13b into the body being probed, i.e. the uterus region of the subject. The pulses are transmitted at a defined frequency over a defined transmit window, or recurrent set of transmit windows. The receive/transmit unit comprises one or more ultrasound transducers for generating and sensing ultrasound signals. It is a form of ultrasound transducer unit, but without comprising signal processing components (which are comprised externally to the unit in this example).

Reflected ultrasound signals are then received back at the ultrasound receive-transmit unit 13. Reflections will be received at the receive-transmit unit at different time points depending upon the depth from which the signal is reflected. As the propagation speed of ultrasound in tissue is known (approximately 1000 meters/second), the time delay between transmission and reception may be mapped to the distance the ultrasound pulse has travelled. This distance is then proportional to the depth.

In some examples, signals are transmitted in a single direction, and ultrasound signals received and gated corresponding to different depths within said single cylindrical beam field. In this way, each ultrasound transducer unit can acquire multiple channels corresponding to different depth regions within the subject

In further examples, the ultrasound transducer unit may comprise an array of individual ultrasound transmitters, and wherein a control means is configured to apply beamforming using the array, to control a directionality of a generated ultrasound beam. The beamforming may be controlled so as to acquire ultrasound data from multiple different beam directions. Signals from a plurality of different depths within each directional beam may be acquired. The ultrasound processing unit may be configured to extract a plurality of different depth channels (according to the procedure described below) from each beam. This approach allows a larger volume of tissue to be scanned with a single transducer unit, and at the same time generate a larger number of ultrasound channels for the PCA algorithm to process.

In either case, the input data may be amplified by an amplifier, and then split into a plurality of separate input channels 52, corresponding to different tissue regions (e.g. different depth or width regions) within the subject.

Signals corresponding to different depths for example may be separated by gating the incoming signal over different temporal receive windows, each gated signal then providing a different input signal channel 52 corresponding to a different depth. Signals corresponding to different laterally displaced regions may be separated through a beam-forming technique such as a delay and sum approach for example.

In some examples, the duration of, and timing between, transit pulses and receive windows can be adjusted so that receive signals from specific desired depths, or different beam field directions, can be obtained, these then being gated over the appropriate time windows to provide different tissue region signals on each of the input signal channels 52.

Pre-processing steps are applied to each input signal channel 52. These may be applied after separating the different input signal channels 52 or before.

In particular, a demodulation and signal integration may be applied to the input signals of each input signal channel 52. Demodulation generates a signal with a frequency equal to the Doppler (frequency) shift of the measured Doppler signal, compared to the original transmitted signal.

Bandpass filtering may be applied to each input signal channel 52. The filtering is configured to select the frequency component of the incoming signal within the frequency range expected for the heartbeat measurement. This ensures only the relevant frequency component of the data is retained, reducing overall noise.

An envelope demodulator may additionally be applied in some examples (not shown). This extracts for each input signal channel 52 an envelope signal corresponding to the change in signal strength (e.g. intensity or variance), as a function of time, for the selected (filtered) frequency range.

The input signal channels are compiled 53 into a single collected set of input signals. A vector may be formed from the set of input signal channels.

The compiled single set 53 of input signal channels are then provided as an input to the principal component analysis algorithm 54.

In summary, this algorithm 54 determines linear combinations (weighted sums) of the input signal channels 52 that capture the largest strength (variance) of the signal, while being statistically uncorrelated. These linear combinations correspond, when composed, to a set of first output signals 56.

The output of the PCA algorithm 54 provides an indication of the total number of heartbeat signal sources present in the collection of input signals 52. However, the first output signals 56 of the PCA may still contain mixtures of the original heartbeat signal sources. The PCA alone therefore may not be sufficient to fully separate the different heartbeat sources.

PCA can be used to reduce the initial number of input channels 52 to the number of uncorrelated, strong pulse rate signals (i.e. the first output signals 56). The set of first output signals 56 typically numbers fewer than the total number of input signal channels 52.

Reducing the number of channels simplifies subsequent processing steps and excludes mathematical subspaces of the compiled input channel vector 53 that contain only noise. The PCA algorithm 54 effectively performs a first stage of unmixing of the different pulse signal sources.

PCA can be performed with a number of algorithms, including, by way of example, online neural network algorithms or other machine learning algorithms. PCA in general may be considered a subset (or a specific technique) of machine learning.

By way of example, chapter 6 ("Principal Component Analysis and Whitening"), in the book "Independent Component Analysis" by Hyvärinen, Karhunen and Oja describes in detail procedures for implementing suitable principal component analysis procedures. The chapter in particular describes several elements of PCA, including one-by-one extraction of principal components and parallel extraction of multiple principal components; sample-by-sample and batch mode algorithms; and methods for determining the number of components that should be extracted.

The first output signals 56 are then provided as an input to a more complex independent component analysis (ICA) procedure 58. This technique determines linear combinations of the first output channels 56 that are statistically independent. This results, in the ideal case, in a number of second output signal channels 60 where each second output signal mostly contains only the Doppler ultrasound signal of one pulse signal source in the abdomen. The ICA algorithm completes the unmixing of the original pulse signal sources.

The PCA algorithm and ICA algorithm will now be explained in more detail.

The PCA algorithm 54 processes the input signal channels 52 and may provide as an output a set of weight vectors (or linear coefficients) that describe how to (linearly) combine the input channels 52 to form the first output channels 56. Preferably, the PCA also outputs the first output channels 56 themselves, which may or may not consist of a smaller number of channels than the set of input channels 52. The PCA may output a vector of the output channels.

In vector/matrix form, PCA computes

$$\mathbf{z}(t) = \mathbf{V} * \mathbf{x}(t),$$

with **x** being a column vector of n elements corresponding to the input signal channels 52, **z** being a column vector of m elements corresponding to the output channels, and **V** being a matrix with *m* rows and *n* columns which makes the elements of **z** statistically uncorrelated and usually also normalizes their statistical variance to 1.

If the output signals are both made uncorrelated and their variance is normalized, the process is otherwise known as "whitening".

PCA algorithms determine a value **of V** and **z**, given **x** as input. There is usually no single unique solution for **V** and **z**; a PCA algorithm finds one of infinitely many possible solutions that make the elements of z uncorrelated. PCA works by considering the variances and the cross-correlations (the so-called second order statistics) of the input signals 52 only, so it can achieve uncorrelatedness, but not complete statistical independence of the elements of **z**.

As a simple example, there may be provided three input channels in **x**, and prior knowledge that there exist a total of two heart rate signal sources present in the channels of **x** at different intensities. The problem is to seek two output channels 56, and **V** in the form of a 2-by-3 matrix.

If the first heart rate signal source is present in the first and second input channel 52 of **x** at equal intensity, and the second heart rate signal source is present only in the third input channel 52 of x, a PCA algorithm should result in a **V** with the form:

$$V = \begin{bmatrix} 1 & 1 & 0 \\ 0 & 0 & 1 \end{bmatrix}$$

In this particular example, the PCA algorithm would in fact also achieve complete separation of the two sources (since they were not really mixed in the first place).

It is noted that as the maximum number of independent signal sources is usually known in fetal monitoring applications, advantageously, this information may be used to reduce the complexity of the PCA procedure, reducing the number of dimensions which the PCA algorithm is required to consider in the processing. The algorithm itself is not required to determine in this case how many output channels it should produce. This may increase the speed of the algorithm.

In particular, due to the matrix operations involved in the algorithm, the computational complexity increases with the third power of the number of channels. Hence, reducing the problem from four (or more) channels to two or three (in case of a twin pregnancy) significantly reduces the computational requirements of the algorithm.

This simplification may be pre-programmed in the algorithm, or it may be provided as an adjustable setting of the algorithm. For instance the processing unit may be configured to receive a user input representative of a total number of heart rate source signals, this being determined for instance based on whether there is a single or double (or more) pregnancy.

PCA is a well-known procedure within the field of signal analysis, and the skilled person will be aware of the principles behind it, and of detailed means for implementing the procedure. By way of example the book, "Handbook of Blind Source Separation" by Comon and Jutten, provides more information on PCA algorithms which may be applied in accordance with embodiments of the present invention. The book "Independent component analysis" by Hyvärinen et al. also contains a chapter dedicated to Principal Component Analysis, which provides detailed explanation on suitable means for implementing PCA algorithms suitable for use in embodiments of the present invention.

The Independent Component Analysis (ICA) algorithm may also output a set of weight vectors (for example represented in matrix form) defining the derived set of linear combinations of the first output signals. It may also output a vector of the second output signals.

In general, an ICA algorithm finds a matrix **W** so that the elements of y(t)

$$y(t) = W * z(t)$$

are statistically independent. z(t) is the output of the PCA/whitening algorithm.

The ICA algorithm looks beyond the second-order statistics considered by the PCA algorithm, and considers further statistical properties such as kurtosis, signal entropy, or mutual information of the channels of y, as these properties quantify statistical dependence/independence.

In general, ICA algorithms may be constructed by choosing a cost function (e.g. kurtosis, signal entropy, or mutual information) that is to be minimized or maximized by finding a suitable **W**, and choosing an optimization algorithm for the minimization/maximization (e.g. gradient descent, stochastic gradient descent, Newton's method).

As the cost function is nonlinear, the algorithm is required to iterate over several approximate solutions in order to find the **W** that minimizes/maximizes the cost function, taking applicable constraints into account (such constraints can be used to prevent the optimization algorithm from simply setting **W** to zero to minimize the cost function for example, or by letting the values of **W** increase without bounds to maximize the cost function).

After application of the PCA and ICA procedures, the matrices **W** and **V** may optionally also be combined into an "unmixing matrix" **B**, where

$$y(t) = W * z(t) = W * V * x(t) = B * x(t)$$

Here, **B** directly describes the linear combinations of **x** that form the output channels of **y**. **B** provides information indicative of whether and how strongly the channels of **x** appear as components of each output channel in **y**.

ICA is a well-known procedure within the field of signal analysis, and the skilled person will be aware of the principles behind it, and of detailed means for implementing the procedure. Further details on example ICA algorithms may be found for example in the book: Appo Hyvärinen, Juha Karhunen, Erkki Oja, "Independent Component Analysis", John Wiley & Sons, Inc., 2001.

The second output signal channels 60 of the ICA may be taken as representative of individual heart rate signal sources.

The second output signals 60 of the ICA may subsequently be provided as an input to a heart rate calculation algorithm. Algorithms for deriving a heart rate measurement or signal based on a Doppler ultrasound signal are known in the art. Some for example are based on autocorrelation.

By way of example, one suitable example heart rate calculation algorithm is outlined in the document US 4,403,184. This example is based on autocorrelation. Using autocorrelation to determine the frequency of a repeating signal is an established technique in the field.

Since each second output signal channel is predominantly composed of only one heart rate signal source, occurrence of erroneous FHR readings due to mixed signals may be very significantly reduced compared to existing solutions.

According to one or more advantageous embodiments, the processing unit may be further configured to derive a physiological source attribution for each of the second output signals 60, i.e. to determine whether each signal corresponds to a maternal heart rate or a fetal heart rate.

This attribution process may be based on a comparative approach comprising comparing one or more properties of the second output signals 60.

For example, properties of the different second output signals 60 such as the average depth of an identified signal source, the pulse rate, the spectral content of the signal, may be compared, and the results used to inform an attribution. There may be stored known average or typical values of one or more of these properties for maternal and fetal heart rate signals respectively, and these used as references to determine an attribution for each of the second output signals 60. Other properties such as maternal ECG or SpO2 pulse rates may additionally be used to inform the attribution process in some examples.

A comparative approach of classifying the signal sources (comparing signal properties of the various output signals 60) is a simpler approach than considering each source in turn and analyzing it to determine an attribution.

Fig. 5 schematically illustrates application of an example embodiment of the invention for detection of two fetal heart rate sources.

Here, as in the example of Fig. 2 described above, the ultrasound beam fields 14a, 14b of a first 12a and second 12b ultrasound transducer unit cross one another. The ultrasound beam field 14a of the first 12a transducer unit contains two fetal heart rate sources 22a, 22b. The ultrasound signal(s) acquired from this beam field therefore contain a mixture of two heart rate sources. The second transducer unit 12b contains only the second fetal heart rate source 22b within its beam field of view 14b. Hence the ultrasound signal channels acquired by these two ultrasound transducers contain a mixture of two heart rate signal sources, and with some of the channels including the same heart rate source.

The ultrasound data from both transducer units 12a, 12b is compiled 53 into the single set of ultrasound signal channels, and the PCA and ICA algorithms 54, 58 successively applied, thereby enabling a set of two second output signal channels to be obtained, one corresponding to the first heart rate source 22a and one to the second heart rate source 22b. A heart rate calculation algorithm may then be applied to these heart rate sources to obtain output heart rate signals 62 corresponding to the first 22a and second 22b fetal heart rate source in the abdomen.

Fig. 6 shows a further example application of an embodiment of the invention.

The two transducer units 12a, 12b in this example record Doppler ultrasound signals from two separate depth ranges ("near" 24a, 24b and "far" 25a, 25b). The three pulse signal sources 22a, 22b and 22c (corresponding to two fetal heart sources, and the maternal heart source respectively) are all present at different intensities in the four input signal channels arriving at the collection point 53. The signal source 22a is present in both channels 24a, 25a of the first transducer unit 12a and in the far channel 25b of the second transducer unit 12b. Signal source 22b is present in the near channel 24b of the second transducer unit 12b. The signal source 22c is present in the far channels 25a, 25b of both transducer units 12a, 12b, at different intensities. The PCA/ICA algorithms 54, 58 perform unmixing of the signal sources from the recorded signal mixtures. Pulse rate calculation is then performed on the reconstructed independent pulse signals, resulting in output pulse signals 1, 2 and M.

The approach employed by embodiments of the present invention carries numerous advantages over known approaches.

In particular, the increased robustness of the signal separation process of the present invention means that fewer constraints are required when positioning the ultrasound transducer units on the maternal abdomen. This renders the electrical fetal monitoring (EFM) system easier and more convenient to use and also improves patient comfort.

Additionally, embodiments of the present invention are able to determine the total number of independent pulse signal sources within the ultrasound field of view, and analyze each of the source signals separately. This can be used to provide both a maternal pulse rate in addition to the fetal pulse rate, or to monitor multiple fetuses with a single transducer.

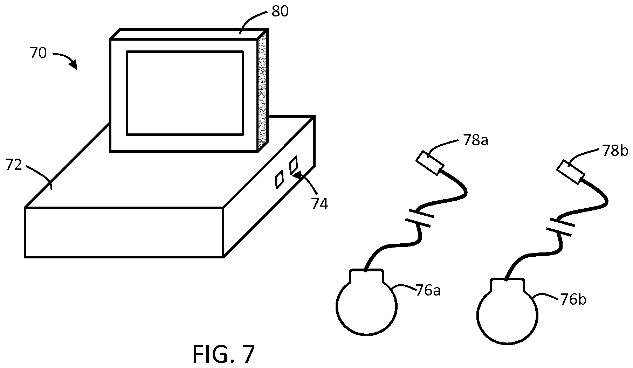

Examples in accordance with a further aspect of the invention provide a patient monitoring system. An example patient monitoring system 70 in accordance with one or more embodiments is shown in Fig. 7.

The patient monitoring system 70 comprises an ultrasound processing unit in accordance with any example or embodiment outlined above or described below, or in accordance with any claim of this application. In the example shown the ultrasound processing unit is incorporated internally within a base station unit 72. In other examples however, the ultrasound processing unit may be incorporated locally within an ultrasound transducer unit 76 with which the base station unit is connected or connectable.

The patient monitoring system 70 further comprises a connection interface in the form of an input connector port 74 for connecting in use to at least two ultrasound transducer units 76a, 76b for receiving the input Doppler ultrasound data, or data derived therefrom. An example set of two ultrasound transducer units 76a, 76b for connecting in use to the base station is shown in Fig. 7. Each transducer unit comprises a respective output connector 78a, 78b shaped to engage with a port of the input connector 74 of the base station.

Where the ultrasound processing unit is included in the base station 72, the input connector 74 may be coupled to the ultrasound processing units to transfer the received ultrasound data.

The connector 74 is shown as a wired connector port in Fig. 7. In other examples, the connector may comprise a wireless connection interface for connecting to wireless ultrasound probes.

The provided patient monitoring system 70 may further include the set of two ultrasound transducer units 76a, 76b coupled to said input connector 74. The transducer units may each be an ultrasound probe for example.

The patient monitoring system in the present example further includes a display 80 operably coupled to the ultrasound processing unit of the base station 72 for displaying results of the analysis procedure performed, e.g. displaying a visual representation of the one or more second output signals.

The patient monitoring system 70 may further include a controller adapted to control acquisition of ultrasound data by a connected transducer unit in use.

The controller may control transmit and receive circuits of the ultrasound transducer unit to acquire the ultrasound signals representative of different tissue regions, e.g. different depths. The controller may control durations of, and timings between, transmit pulses and receive windows. The controller may control gating of the input Doppler signal data over defined time windows to thereby separate different input signal channels corresponding to different tissue regions within the subject's tissue.

In some examples said controller may be comprised locally within the ultrasound transducer unit, or the control steps performed by it may be performed locally at the ultrasound transducer unit.

As mentioned above, one of the ultrasound transducer units may comprise the ultrasound processing unit. It may be an ultrasound probe unit for instance incorporating one or more ultrasound transducers and an ultrasound processing unit operatively coupled with the processing unit. The ultrasound transducer unit may locally perform at least a subset of the ultrasound data pre-processing steps and/or control steps described above.

The patient monitoring system may take different forms to that described above. For example the patient monitoring system may comprise a monitoring station (e.g. a trolley-type monitoring station), comprising a display, and being connectable with a plurality of ultrasound transducer units.

In any example, the patient monitoring system may be connectable with any number of further sensors or data sources for monitoring the same patient or different patients.

Examples in accordance with a further aspect of the invention provide an ultrasound apparatus comprising: an ultrasound processing unit in accordance with any example or embodiment outlined above or described below, or in accordance with any claim of this application; and one or more ultrasound transducers, operatively coupled to the ultrasound processing unit, for providing at least one of the first and second input Doppler ultrasound data to the ultrasound processing unit.

The apparatus may for example comprise an ultrasound probe unit, the probe unit incorporating the ultrasound processing unit and at least a portion of the one or more ultrasound transducers. For instance, the probe may comprise a housing incorporating the one or more ultrasound transducers and the ultrasound processing unit.

Examples in accordance with a further aspect of the invention provide an ultrasound processing method for use in distinguishing different heart rate sources within received Doppler ultrasound data, the method comprising:

> receiving 32 first input Doppler ultrasound data from a first ultrasound transducer source, and receiving second input Doppler ultrasound data from a second ultrasound transducer source;
> compiling 34 from the first and second input Doppler ultrasound data a single set of input ultrasound signal channels, each corresponding to a different particular tissue region within the subject;
> performing 36 a principal component analysis, PCA, procedure, configured to identify one or more linear combinations of the input signal channels which are statistically uncorrelated, the linear combinations defining, when composed, a set of first output signals, and
> performing 38 an independent component analysis, ICA, procedure, configured to identify one or more linear combinations of said first output signals which are statistically independent from one another, said one or more linear combinations defining a set of one or more second output signals, the one or more second output signals providing signals corresponding to distinct heart rate sources.

Implementation options and details for each of the above steps may be understood and interpreted in accordance with the explanations and descriptions provided above for the apparatus aspect of the present invention (i.e. the ultrasound processing unit aspect).

Any of the examples, options or embodiment features or details described above in respect of the apparatus aspect of this invention (in respect of the ultrasound processing unit) may be applied or combined or incorporated into the present method aspect of the invention.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. An ultrasound processing unit, for use in fetal monitoring for distinguishing different heart rate sources within received Doppler ultrasound data, the ultrasound processing unit being communicatively coupleable in use with at least two ultrasound transducer sources; and the ultrasound processing unit configured to:

   receive (32) first input Doppler ultrasound data from a first ultrasound transducer source, and receive second input Doppler ultrasound data from a second ultrasound transducer source; compile (34) from the first and second input Doppler ultrasound data a single set of input ultrasound signal channels, each corresponding to a different particular tissue region within the subject;

   perform (36) a principal component analysis, PCA, procedure, configured to identify one or more linear combinations of the input signal channels which are statistically uncorrelated, the linear combinations defining, when composed, a set of first output signals, and perform (38) an independent component analysis, ICA, procedure, configured to identify one or more linear combinations of said first output signals which are statistically independent from one another, said one or more linear combinations defining a set of one or more second output signals, the one or more second output signals thereby providing signals corresponding to distinct heart rate sources.

2. The ultrasound processing unit as claimed in claim 1, wherein the compiled set of ultrasound signal channels comprises channels corresponding to at least two different depth regions within the subject, and/or comprises channels corresponding to at least two different lateral regions within the subject.

3. The ultrasound processing unit as claimed in claim 1 or 2, wherein the PCA procedure is configured to identify the linear combinations of said input signal channels that result in first output signals having a combined signal strength or variance exceeding a defined threshold while being statistically uncorrelated with one another.

4. The ultrasound processing unit as claimed in any of claims 1-3, wherein the processing unit is further configured to generate the set of second output signals in accordance with the identified linear combinations.

5. The ultrasound processing unit as claimed in claim 4, wherein the processing unit is adapted to process the second output signals to derive from each a heart rate signal or heart rate measurement.

6. The ultrasound processing unit as claimed in any of claims 1-5, wherein the ultrasound processing unit is further adapted to attribute to each of the second output signals a physiological source.

7. The ultrasound processing unit as claimed in any of claims 1-6, wherein the compiling comprises compiling from the first and second input Doppler ultrasound data a single vector of input ultrasound signal channels, each corresponding to a different particular tissue region within the subject.

8. An ultrasound apparatus comprising:

   at least one ultrasound processing unit as claimed in any of claims 1-7; and
   one or more ultrasound transducers, operatively coupled to the ultrasound processing unit, for providing at least one of the first and second input Doppler ultrasound data to the ultrasound processing unit.

9. The ultrasound apparatus as claimed in claim 8, wherein the ultrasound apparatus comprises an ultrasound probe unit, the probe unit incorporating the ultrasound processing unit and at least a portion of the one or more ultrasound transducers.

10. A patient monitoring system comprising:

   an ultrasound processing unit as claimed in any of claims 1-7; and
   a connection interface for connecting in use to at least two ultrasound transducer units.

11. The patient monitoring system as claimed in claim 10, further comprising a set of at least two ultrasound transducer units coupled to said connection interface.

12. The patient monitoring system as claimed in claim 10 or 11, further including a controller adapted to control acquisition of ultrasound data by at least one connected ultrasound transducer unit in use.

13. An ultrasound processing method for use in distinguishing different heart rate sources within received Doppler ultrasound data, the method comprising:

   receiving (32) first input Doppler ultrasound data from a first ultrasound transducer source, and receiving second input Doppler ultrasound data from a second ultrasound transducer source;
   compiling (34) from the first and second input Doppler ultrasound data a single set of input ultrasound signal channels, each corresponding to a different particular tissue region within the subject;
   performing (36) a principal component analysis, PCA, procedure, configured to identify one or more linear combinations of the input signal channels which are statistically uncorrelated, the linear combinations defining, when composed, a set of first output signals, and
   performing (38) an independent component analysis, ICA, procedure, configured to identify one or more linear combinations of said first output signals which are statistically independent from one another, said one or more linear combinations defining a set of one or more second output signals, the one or more second output signals thereby providing signals corresponding to distinct heart rate sources.

14. The method as claimed in claim 13, wherein the compiled set of ultrasound signal channels comprises channels corresponding to at least two different depth regions within the subject, and/or comprises channels corresponding to at least two different lateral regions within the subject.

FIG. 1

FIG. 2

FIG. 3

FIG. 4

FIG. 5

FIG. 6

FIG. 7

| | Europäisches Patentamt<br>European Patent Office<br>Office européen des brevets | **EUROPEAN SEARCH REPORT** | Application Number<br>EP 19 17 0882 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KRIBECHE A ET AL: "Separating fetal doppler signals in pregnancy using independent component analysis : application to the extraction of fetal heart rate and global movement", ULTRASONICS SYMPOSIUM, 2005 IEEE ROTTERDAM, THE NETHERLANDS 18-21 SEPT. 2005, PISCATAWAY, NJ, USA,IEEE, vol. 2, 18 September 2005 (2005-09-18), pages 1319-1322, XP010899087, DOI: 10.1109/ULTSYM.2005.1603096 ISBN: 978-0-7803-9382-0 * the whole document * | 1-14 | INV.<br>A61B8/02<br>A61B8/08 |
| A | US 2005/267376 A1 (MAROSSERO DOROTHEE [US] ET AL) 1 December 2005 (2005-12-01) * paragraphs [0089] - [0098] * | 1-14 | |
| A | US 2016/022164 A1 (BROCKWAY MARINA [US] ET AL) 28 January 2016 (2016-01-28) * paragraphs [0001], [0005], [0011] - [0013], [0099] * | 1-14 | |
| A | CN 109 199 375 A (UNIV SOUTHEAST) 15 January 2019 (2019-01-15) * the whole document * | 1-14 | TECHNICAL FIELDS SEARCHED (IPC)<br>A61B |
| A | US 2016/270670 A1 (OZ OREN [IL] ET AL) 22 September 2016 (2016-09-22) * paragraphs [0216], [0376], [0396], [0497], [0505] * | 1-14 | |
| | -/-- | | |

The present search report has been drawn up for all claims

| Place of search | Date of completion of the search | Examiner |
|---|---|---|
| Munich | 28 October 2019 | Küster, Gunilla |

EPO FORM 1503 03.82 (P04C01)

page 1 of 2

Europäisches
Patentamt
European
Patent Office
Office européen
des brevets

# EUROPEAN SEARCH REPORT

Application Number

EP 19 17 0882

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SOPHIE RIBES ET AL: "Multidimensional Ultrasound Doppler Signal Analysis for Fetal Activity Monitoring", ULTRASOUND IN MEDICINE AND BIOLOGY., vol. 41, no. 12, 1 December 2015 (2015-12-01), pages 3172-3181, XP055635778, US ISSN: 0301-5629, DOI: 10.1016/j.ultrasmedbio.2015.07.026 * section "Materials" and fig. 1 on p. 3173, section "Dimension reduction and sparse blind source separation" on p. 3174, Appendix B * | 1-14 | |
| A | US 2007/260155 A1 (RAPOPORT EZRA J [US] ET AL) 8 November 2007 (2007-11-08) * paragraphs [0014], [0021], [0084] - [0087], [0104] - [0114] * | 1-14 | |

TECHNICAL FIELDS
SEARCHED       (IPC)

The present search report has been drawn up for all claims

| Place of search | Date of completion of the search | Examiner |
|---|---|---|
| Munich | 28 October 2019 | Küster, Gunilla |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 17 0882

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2019

| Patent document cited in search report | | Publication date | Patent family member(s) | | Publication date |
|---|---|---|---|---|---|
| US 2005267376 | A1 | 01-12-2005 | EP 1776041 A2 | | 25-04-2007 |
| | | | US 2005267376 A1 | | 01-12-2005 |
| | | | US 2005267377 A1 | | 01-12-2005 |
| | | | WO 2005117692 A2 | | 15-12-2005 |
| US 2016022164 | A1 | 28-01-2016 | NONE | | |
| CN 109199375 | A | 15-01-2019 | NONE | | |
| US 2016270670 | A1 | 22-09-2016 | US 2016270670 A1 | | 22-09-2016 |
| | | | US 2017172426 A1 | | 22-06-2017 |
| | | | US 2019076030 A1 | | 14-03-2019 |
| US 2007260155 | A1 | 08-11-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4403184 A

**Non-patent literature cited in the description**

- **APPO HYVÄRINEN ; JUHA KARHUNEN ; ERKKI OJA.** Independent Component Analysis. John Wiley & Sons, Inc, 2001

- **HYVÄRINEN.** Independent component analysis